# FASCICULE DE BREVET EUROPEEN

(11) **EP 1 335 701 B1**
(45) Date de publication et mention de la délivrance du brevet: **14.06.2006**
(21) Numéro de dépôt: 01982561.1
(22) Date de dépôt: 26.10.2001
(51) Int. Cl.: A61K 8/92, A61K 8/97, A61Q 19/06

(54) **COMPOSITION ANTI-ADIPEUSE A BASE D'EXTRAITS DE BULBES D'AIL ET UTILISATIONS COSMETIQUES ET THERAPEUTIQUES**
MITTEL GEGEN FEETGEWEBE ENTHALTEND KNOBLAUCH ZWIEBELGEWÄCHSE
ANTI-ADIPOSIS TOPICAL TREATMENT COMPOSITION BASED ON GARLIC BULB EXTRACTS

(30) Priorité: 31.10.2000 FR 0013973
(43) Date de publication de la demande: 20.08.2003
(73) Titulaire: Pierre Fabre Dermo-Cosmetique, 92100 Boulogne (FR)
(72) Inventeur: TOMATIS, Isabelle, F-31170 Tournefeuille (FR)
(74) Mandataire: Cabinet BARRE LAFORGUE & associés
(86) Numéro de dépôt international: PCT/FR2001/003325
(87) Numéro de publication internationale: WO 2002/036093

(56) Documents cités:
- EP-A- 0 333 548
- EP-A- 0 449 717
- EP-A- 0 503 852
- EP-A- 0 923 937
- DE-A- 19 858 670
- FR-A- 2 804 319
- GB-A- 842 404
- GB-A- 1 106 551
- PATENT ABSTRACTS OF JAPAN vol. 017, no. 185 (C-1047) & JP 04 338336 A (RIKEN HERUSU KK) cité dans la demande
- DATABASE CAPLUS [en ligne] CHEMICAL ABSTRACTS SERVICE, COLUMBUS, OHIO, US; retrieved from STN Database accession no. 2000:114025 XP002173644
- DATABASE WPI Week 200043 Derwent Publications Ltd., London, GB; AN 2000-489113 XP002173645 & KR 99 055 308 A (LG CHEM), 15 juillet 1999 (1999-07-15)

## Description

L'invention concerne une composition de traitement topique anti-adipeux, et les utilisations de cette composition et/ou d'extraits de bulbes d'ail à titre cosmétique, pour le traitement préventif ou curatif de la cellulite et des surcharges adipeuses dermiques localisées, ou à titre thérapeutique pour le traitement de l'obésité.

Plus de 90 % de femmes ont de la cellulite à un degré plus ou moins important. Les causes de la cellulite sont nombreuses : prédispositions génétiques, dérèglement hormonal, erreurs diététiques, problèmes circulatoires, stress ou déprime. De façon schématique, la cellulite correspond à l'accentuation du tissu adipeux dans certaines régions du corps, en particulier sur les hanches, les fesses, les genoux et les avant-bras. Ce tissu adipeux ou masse grasse de l'organisme est présent sous pratiquement toute la surface de la peau. La cellulite est souvent associée à une surcharge adipeuse globale et la stabilité de la masse grasse est un point important dans le contrôle du développement de la cellulite.

La cellulite est localisée au niveau du derme. Dans l'hypoderme, couche profonde du derme, les cellules adipeuses, ou adipocytes, se réunissent pour former des lobules délimités par des cloisons (travées de fibres de collagène) parallèles entre elles et perpendiculaires à la surface cutanée. L'adipocyte est une grosse cellule dont 80 % du volume est constitué par une ou plusieurs vacuoles de lipides. Sa grosseur est variable. Si la vacuole est surchargée de graisse, le volume de l'adipocyte s'accroît et le tissu conjonctif dermique s'épaissit.

La cellulite dépend aussi de critères fonctionnels, car elle est aussi un amas localisé, associé à une insuffisance du tonus veineux et/ou à une altération du système lymphatique.

La première déformation du tissu cellulitique s'effectue au niveau des adipocytes. Dès que le volume des adipocytes augmente, les cloisons vont subir un étirement profond qui apparaît à la surface de l'épiderme sous la forme de dépressions à certains endroits. Ces dépressions apparaissent encore plus si la peau est pincée : c'est la peau d'orange.

La deuxième déformation se situe au niveau de la substance fondamentale. La substance fondamentale se modifie selon la quantité d'eau qu'elle retient. C'est ce qui explique la perte de souplesse de la peau sur une localisation cellulitique. La pression d'eau retenue par la substance fondamentale va écraser les cellules et par la-même entraîner la dégradation des fibres d'élastine et de collagène.

Enfin, la dernière conséquence d'une rétention d'eau excessive dans la substance fondamentale est une compression des vaisseaux sanguins et lymphatiques. Cette compression a pour conséquence une diminution du débit sanguin veineux et du débit de la lymphe qui est ressentie aussi bien au plan de l'épiderme qu'à celui de l'hypoderme. Il en résulte un mauvais équilibre sanguin et une élimination déficiente des toxines et des déchets.

D'un point de vue métabolique, dans le tissu adipeux, l'adipocyte fonctionne comme un réservoir d'énergie pour l'organisme.

Pour assurer sa fonction de réservoir d'énergie, l'adipocyte est capable de stocker l'énergie sous forme de triglycérides dans ses gouttelettes lipidiques (c'est la lipogénèse) puis de libérer cette énergie sous la forme d'acides gras dans la circulation sanguine (c'est la lipolyse).

C'est l'équilibre entre ces deux voies métaboliques (lipogénèse et lipolyse) qui conditionne l'adiposité.

La fonction lipogénique de l'adipocyte nécessite la présence d'acides gras essentiellement issus des lipoprotéines circulantes riches en triglycérides.

Sur le versant catabolique, la lipolyse correspond à la séparation des constituants des triglycérides adipocytaires en glycérol d'une part, et en acides gras d'autre part. Cette lipolyse se fait sous l'action d'une enzyme limitante, la lipase hormono-sensible. L'activation de la lipase hormono-sensible dépend de la teneur en AMP cyclique. Dans le tissu adipeux humain, les hormones et divers agents sont capables de moduler les concentrations en AMPc intracellulaire, et participent donc au contrôle de la lipase hormono-sensible donc à la lipolyse.

Actuellement, toutes les crèmes amincissantes connues contiennent au moins un agent actif qui favorise la lipolyse et/ou inhibe la lipogénèse, ou qui vise à restaurer la microcirculation capillaire et lymphatique.

Toutefois, malgré la diversité des crèmes amincissantes mises au point à l'heure actuelle et le grand nombre de thérapies annexes proposées dans le traitement de la cellulite (stimulation électrique, chaleur, massages ....), aucun traitement ne s'avère réellement efficace pour lutter contre la cellulite.

Parallèlement, depuis quelques années on a mis en évidence les mécanismes impliqués dans l'expansion de la masse grasse.

Le développement excessif de la masse adipeuse est habituellement lié à une augmentation du volume des adipocytes, et de leur contenu en triglycérides sans qu'il y ait une élévation de leur nombre : on parle d'hypertrophie. Dans certains cas d'excès pondéral, l'hypertrophie s'accompagne d'une hyperplasie, c'est-à-dire d'une augmentation du nombre des cellules adipeuses.

Il est aussi maintenant démontré que le nombre de cellules graisseuses n'est pas déterminé en période périnatale, la formation des adipocytes pouvant s'effectuer tout au long de la vie. Elle se réalise à partir de cellules précurseurs, les préadipocytes. Le préadipocyte est une cellule mince, à morphologie fibroblastique : c'est lui et non l'adipocyte, qui est capable de se multiplier.

Schématiquement, la différenciation terminale adipocytaire se fait en deux étapes avec transformation du préadipocyte en adipocyte mature, ce dernier étant capable d'accumuler des triglycérides.

Toutefois, bien que de telles constatations ouvrent une voie nouvelle intéressante pour le traitement de la cellulite, elles n'ont pas été suivies d'effet dans la pratique faute d'avoir trouvé des principes actifs permettant par application topique d'entraîner une inhibition de la conversion adipocytaire des préadipocytes en adipocytes matures.

La présente invention vise à combler cette lacune et a pour principal objectif de fournir une composition thérapeutique ou cosmétique pour le traitement par application topique anti-adipeux en usage externe (sur la peau), agissant notamment de façon très efficace sur le blocage de la différenciation des préadipocytes en adipocytes.

Plus particulièrement, l'invention vise à proposer un traitement amélioré, cosmétique ou thérapeutique, de la cellulite, des surcharges adipeuses dermiques localisées, et de l'obésité.

L'invention vise de surcroît à proposer de nouvelles utilisations à titre cosmétique ou thérapeutique, d'extraits -notamment d'extraits non aqueux- de bulbes d'Allium sativum (ail).

Dans tout le texte, le terme "bulbes" englobe aussi bien des bulbes de la plante (groupes de gousses d'ail) que des gousses d'ail séparées les unes des autres, pelées ou non.

L'invention vise aussi à proposer de nouvelles compositions à base d'extraits de bulbes d'ail et leurs applications, notamment à des fins cosmétiques ou thérapeutiques.

Un autre objectif de l'invention est de fournir une composition thérapeutique agissant sur des adipocytes hypertrophiés matures dans le cadre du traitement de l'obésité.

Dans toute la suite, les termes définissant les extraits (extrait, huile essentielle, absolue, concrète, oléorésine ...) sont utilisés selon la terminologie définie par la norme NF T 75-006 (Février 1998).

L'invention concerne donc une composition de traitement topique à usage externe telle que définie par la revendication 1. Une composition selon l'invention est caractérisée en ce qu'elle est constituée :
- d'une quantité efficace d'au moins un agent actif anti-adipeux choisi parmi les extraits de bulbes d'Allium sativum à l'exception d'une oléorésine d'extraction extraite à l'hexane,
- optionnellement, d'un ou plusieurs autres agents actifs complémentaires, à l'exception des extraits de Nympheaceae,
- d'un excipient cosmétiquement ou pharmaceutiquement acceptable et adapté pour une application topique à usage externe -notamment sur la peau-.

Avantageusement, la composition selon l'invention comprend une quantité d'au moins un agent actif anti-adipeux adaptée pour au moins sensiblement inhiber la différenciation des préadipocytes en adipocytes matures, sans sensiblement provoquer d'irritation ou de sensibilisation cutanée.

Avantageusement et selon l'invention, la composition comprend une quantité efficace d'au moins un agent actif anti-adipeux choisi parmi les extraits non aqueux de bulbes d'Allium sativum.

Avantageusement et selon l'invention la quantité d'extrait(s) de bulbes d'Allium sativum est comprise entre 3 ppm et 20 ppm -notamment de l'ordre de 10 ppm-.

Dans un premier mode de réalisation, et selon l'invention, la composition comprend à titre d'agent actif anti-adipeux, une huile essentielle de bulbes d'Allium sativum et un agent émulsionnant.

Par ailleurs, en vue de la réalisation d'une crème applicable par voie topique, et de façon avantageuse, le mélange huile essentielle / agent émulsionnant est incorporé dans un gel et un excipient liquide tel que l'eau.

Dans un deuxième mode de réalisation, et selon l'invention, la composition comprend à titre d'agent actif anti-adipeux, au moins une absolue de bulbes d'Allium sativum en solution aqueuse. Avantageusement et selon l'invention, elle comprend au moins une absolue obtenue à partir d'une concrète extraite de bulbes d'Allium sativum. Avantageusement et selon l'invention, elle comprend en outre au moins une absolue obtenue à partir d'une oléorésine d'extraction extraite de bulbes d'Allium sativum.

Plus particulièrement, une composition selon l'invention est avantageusement caractérisée en ce qu'elle comprend, à titre d'agent actif anti-adipeux, au moins un extrait de bulbes d'Allium sativum choisi parmi une huile essentielle, une absolue obtenue à partir d'une concrète extraite à l'hexane, une absolue obtenue à partir d'une concrète extraite à l'acétate d'éthyle, une absolue obtenue à partir d'une oléorésine d'extraction extraite à l'acétone.

Selon l'invention, pour une composition comprenant une absolue obtenue à partir d'une concrète extraite à l'hexane, la quantité de celle-ci est avantageusement comprise entre 3 ppm et 20 ppm -notamment de l'ordre de 10 ppm-.

Selon l'invention, pour une composition comprenant au moins un extrait de bulbes d'Allium sativum choisi parmi une huile essentielle, une absolue obtenue à partir d'une concrète extraite à l'acétate d'éthyle, une absolue obtenue à partir d'une oléorésine d'extraction extraite à l'acétone, la quantité d'extrait(s) est avantageusement comprise entre 5 ppm et 20 ppm -notamment de l'ordre de 10 ppm-.

Il est à noter que dans la pratique de la présente invention, bien que les différentes absolues soient diluées à 25% dans l'éthanol, la proportion d'absolue(s) en ppm des différentes compositions selon l'invention se réfère quant à elle à une quantité d'absolue(s) "pure(s)", c'est-à-dire une absolue contenant moins de 2% d'éthanol résiduel.

L'invention se fonde ainsi sur la découverte très étonnante et inattendue que des extraits de bulbes d'Allium sativum présentent une activité très efficace de blocage de la différenciation des cellules adipeuses.

Il est à noter que l'Allium sativum est réputé depuis de nombreux siècles, comme ayant une action antiseptique, anti-virale, cardio-protectrice, tonique, diurétique ....

EP-0923 937 décrit une composition de traitement des désordres de la peau tels qu'allergies, dépigmentations, mycoses, gerçures, hyperkératoses, coupures, brûlures, comprenant au moins deux extraits de plantes sous forme d'huile ou de poudre obtenus par extraction aux solvants à partir de plantes séchées. Il s'agit donc d'oléorésines d'extraction (ou résinoïdes). Parmi les différents extraits, la composition comprend 1 à 3% d'une oléorésine d'extraction d'ail extraite à l'hexane, qui ne présente pas d'activité anti-adipeuse.

GB-1106551 décrit l'utilisation d'extraits de plantes de la famille des Nympheaceae à titre d'agents actifs pour le traitement thérapeutique des néoplasmes et de la cellulite. Les compositions décrites incluent aussi une ou plusieurs huile(s) essentielle(s) complémentaire(s) de plantes d'autres espèces, notamment de la famille des Cruciferaceae (par exemple l'huile de moutarde ou l'huile de raifort) ou de la famille des Liliaceae (par exemple l'huile d'ail), produisant un effet de stabilisation des extraits de Nympheaceae, et un effet synergique avec ces extraits. Pour le traitement de la cellulite par voie topique, les huiles essentielles sont dissoutes dans l'huile d'olive. Ce document indique que lesdites huiles essentielles complémentaires n'ont aucun effet propre isolément.

EP-O333548 décrit des émulsions aqueuses stables d'huile essentielle d'orange comprenant 1 à 45 parties en poids d'huile essentielle ; 0,01 à 1 partie en poids d'un émulsifiant non-ionique choisi parmi les sucroglycérides et les sucroesters ; 0,2 à 1,5 parties en poids de gomme xanthane, et le complément en poids d'eau. Ces émulsions peuvent être utilisées pour la préparation de concentrats d'aromatisation de produits alimentaires, notamment de boissons sans alcool et de confiserie ou de concentrats pour cosmétiques. Ce document indique théoriquement que toutes les "essences" de plantes (en fait des extraits quelconques) peuvent être utilisées dans l'émulsion, et cite donc parmi elles notamment "l'essence d'ail". Néanmoins, il ne donne des modes de réalisation que dans le cas de l'essence d'orange. Or, avec les proportions indiquées, une émulsion stable d'huile essentielle ne peut pas être obtenue avec toutes les huiles essentielles connues, et en particulier avec l'huile essentielle d'ail. Ce document ne procure donc pas d'enseignement réaliste relatif à une composition à base d'huile essentielle d'ail.

JP-04 338 336 décrit des comprimés de traitement de l'obésité par voie orale comprenant des extraits d'ail obtenus à partir d'ail séché et d'une extraction à l'eau ou à l'éthanol. Ce document vise donc à valoriser l'effet hypolipidémique bien connu de certains extraits d'ail dans le sang circulant.

FR-2804319 publié le 3 août 2001 décrit une composition cosmétique amincissante comprenant un extrait de plante contenant de l'ANP, obtenu nécessairement par extraction à l'eau et à partir des tiges, feuilles et pétales des plantes. Ce document cite diverses plantes utilisables, dont l'ail, mais ne décrit pas une composition comprenant un extrait de bulbes d'Allium sativum. Le mécanisme d'action de la composition consiste à favoriser la lipolyse des adipocytes, mais n'agit en aucun cas sur la transformation des préadipocytes en adipocytes matures.

Ainsi, rien dans les connaissances actuelles ne laissait présager que l'utilisation d'extraits de bulbes d'Allium sativum pourrait conduire à l'émergence d'une nouvelle génération de crèmes amincissantes agissant non plus seulement sur les mécanismes de stockage/déstockage des graisses (donc lipogénèse/lipolyse), mais notamment sur le processus de différenciation des préadipocytes en adipocytes matures.

Les extraits d'Allium sativum selon l'invention agissent localement sur la cellule adipeuse sans entraîner la libération dans la circulation sanguine d'une partie de ses acides gras qui, faute d'être brûlés, pourraient s'avérer préjudiciables en terme de santé (dépôt sur la paroi artérielle ou le tissu intra-abdominal).

Donc, les extraits d'Allium sativum selon l'invention agissent sur un versant nouveau de la lutte contre la cellulite : en limitant le recrutement de préadipocytes dormants ils limitent l'expansion du tissu adipeux.

Mais, ce n'est pas là leur seule efficacité, car il s'avère qu'avec un seul agent actif anti-adipeux selon l'invention, plusieurs clés pour lutter contre la cellulite sont fournies. En effet, un agent anti-adipeux selon l'invention assure aussi une bonne vascularisation du tissu adipeux en réduisant les taux en agents vasopresseurs.

Les extraits d'Allium sativum selon l'invention constituent donc des agents actifs anti-adipeux présentant une efficacité maximale rendant inutile de multiplier les combinaisons d'actifs dans les formulations cosmétiques ou thérapeutiques amincissantes.

D'autres expérimentations ont également permis de constater, de façon inattendue, que les agents actifs anti-adipeux selon l'invention sont aussi actifs au niveau d'adipocytes hypertrophiés matures. Ils agissent donc sur les deux processus de croissance adipeuse, hyperplasie et hypertrophie, ce qui permet de les utiliser avec succès dans le domaine thérapeutique pour le traitement de l'obésité.

Un autre avantage constaté des agents actifs anti-adipeux selon l'invention réside dans le fait que les phénomènes obtenus sont réversibles et que par conséquent ils ne conduisent pas à un blocage du mécanisme physiologique.

Par ailleurs, l'efficacité des agents actifs anti-adipeux selon l'invention reposant notamment sur la diminution du recrutement des pré-adipocytes dormants, les compositions thérapeutiques ou cosmétiques selon l'invention constituent un soin du corps, qui de façon préférentielle, est appliqué en traitement externe sur les zones de la peau à traiter au moins une fois par jour, tous les jours de l'année.

Dans ce cas, la composition selon l'invention ne nécessite aucun autre agent actif que celui selon l'invention, et le (les) extrait(s) d'Allium sativum peut (peuvent) constituer le (les) seul(s) agent(s) actif(s) anti-adipeux de ladite composition. Dès lors, la composition selon l'invention est exempte d'agent actif complémentaire. En particulier, de préférence 1' (les) extrait(s) de bulbes d'Allium sativum est (sont) le (les) seul(s) extrait(s) de plante d'une composition selon l'invention.

Toutefois, une composition selon l'invention peut également être utilisée pour des soins dits "flash" consistant à effectuer un traitement durant 2 ou 3 mois, notamment avant la période estivale. Dans ce cas, elle comprend avantageusement un agent actif complémentaire consistant en un agent lipolytique de tout type connu en soi. Avantageusement une composition selon l'invention comprend au moins un agent lipolytique choisi parmi les agents lipolytiques d'origine naturelle (tel que la caféine et les agents lipolytiques) de synthèse (composé(s) de bases xanthiques), capables de stimuler la synthèse d'AMPc.

D'autres agents actifs complémentaires, notamment à effet cosmétique, peuvent être associés aux agents actifs anti-adipeux selon l'invention.

Une composition selon l'invention peut contenir un certain nombre d'agents capables de : stabiliser le réseau élastique (par exemple des dérivés de silicium) ; et/ou procurer un effet tenseur immédiat (incorporation de polymères de synthèse ou de protéines végétales (soja, germe de blé) ; et/ou assurer une hydratation optimale (incorporation d'agents humectants et/ou d'agents restructurant).

Avantageusement, une composition selon l'invention ne nécessite pas l'incorporation d'agents susceptibles d'améliorer son pouvoir de pénétration (tels que des exfoliants).

De même, il n'est pas nécessaire d'incorporer à une composition selon l'invention des agents susceptibles d'améliorer la microcirculation cutanée.

La composition selon l'invention peut comprendre tout excipient compatible avec les agents actifs anti-adipeux selon l'invention, acceptable pharmaceutiquement ou cosmétiquement, et pouvant comprendre tous adjuvants appropriés y compris gélifiants, parfums, conservateurs, stabilisants, colorants ... permettant d'obtenir la forme galénique souhaitée et appropriée. Une composition selon l'invention peut être exempte de gomme xanthane.

Dans le cas d'une huile essentielle de bulbes d'Allium sativum on peut utiliser, à titre d'agent émulsionnant, notamment le produit désigné Liposol commercialisé par la société SIEGEN (Genève, Suisse), ou tout autre émulsionnant, notamment tout émulsionnant qui n'est ni un sucroglycéride ni un sucroester.

La quantité d'émulsionnant utilisée est adaptée pour obtenir l'émulsion souhaitée. Elle est en pratique beaucoup plus importante -typiquement 50 fois- que celle d'huile essentielle.

Dans le cas d'une absolue en solution aqueuse, la composition selon l'invention se présente avantageusement sous la forme d'une simple lotion.

Des exemples de réalisation d'une composition selon l'invention sont les suivants.

Pour la réalisation d'une crème applicable par voie topique, l'huile essentielle d'Allium sativum est en premier lieu incorporée dans un agent émulsionnant tel que du Liposol (SIEGEN, Suisse), les proportions respectives en volume étant par exemple de l'ordre de 0,001 % d'huile essentielle, de 0,05 % de Liposol, et du complément à 100 % d'excipient.

Avec les absolues on peut préparer par exemple des lotions. A titre d'exemples, les quantités en concentration en volume des divers constituants peuvent être les suivants :
- composition n° 1 :
   - Absolue de bulbes d'Allium sativum obtenue à partir d'une concrète extraite à l'hexane : 0,0003 % à 0,002 %
   - Excipient : concentration adaptée pour obtenir 100%
- Composition n° 2 :
   - Absolue de bulbes d'Allium sativum obtenue à partir d'une concrète extraite à l'acétate d'éthyle : 0,0005 % à 0,002 %
   - Excipient : concentration adaptée pour obtenir 100%
- Composition n° 3 :
   - Absolue de bulbes d'Allium sativum obtenue à partir d'une oléorésine d'extraction extraite à l'acétone : 0,0005 % à 0,002 %
   - Excipient : concentration adaptée pour obtenir 100%.

Une composition selon l'invention est extrêmement simple et peu coûteuse.

L'invention s'étend à un procédé de traitement cosmétique et/ou thérapeutique préventif ou curatif de la cellulite, des surcharges adipeuses dermiques localisées ou de l'obésité, caractérisé en ce qu'on applique par voie topique sur des zones de peau à traiter au moins un extrait de bulbes d'Allium sativum à titre d'agent actif anti-adipeux. Par traitement des surcharges adipeuses dermiques, on entend un traitement de la masse graisseuse localisée aussi bien au niveau du derme que de l'hypoderme.

L'invention concerne aussi un procédé de traitement caractérisé en ce qu'on utilise une composition selon l'invention.

L'invention concerne aussi l'utilisation d'au moins un extrait de bulbes d'Allium sativum à titre d'agent actif anti-adipeux pour le traitement cosmétique préventif ou curatif, par application topique sur des zones de peau à traiter, de la cellulite et des surcharges adipeuses dermiques localisées.

L'invention concerne aussi l'utilisation d'au moins un extrait de bulbes d'Allium sativum à titre d'agent actif anti-adipeux pour la préparation d'une composition thérapeutique pour le traitement préventif ou curatif, par application topique sur des zones de peau à traiter (ventre, cuisses, hanches, fessiers, bourrelets, membres ...) de l'obésité. L'invention concerne aussi un procédé de préparation d'une composition thérapeutique pour le traitement préventif ou curatif, par application topique sur des zones de peau à traiter, de l'obésité, caractérisé en ce qu'on incorpore dans la composition une quantité efficace d'au moins un extrait de bulbes d'Allium sativum à titre d'agent actif anti-adipeux.

Avantageusement, dans un procédé ou une utilisation selon l'invention, on utilise une quantité d'au moins un extrait de bulbes d'Allium sativum adaptée pour entraîner au moins sensiblement une inhibition de la différenciation des préadipocytes en adipocytes matures et/ou pour au moins sensiblement limiter ou inhiber l'hypertrophie des adipocytes matures, et ce sans provoquer d'irritation ni de sensibilisation cutanée.

L'invention s'étend aussi à un procédé d'inhibition extra-corporelle de la conversion adipocytaire des préadipocytes en adipocytes matures, caractérisé en ce qu'on met une quantité efficace d'au moins un extrait de bulbes d'Allium sativum au contact des préadipocytes ou d'un tissu biologique les incorporant.

Avantageusement, dans un procédé ou une utilisation selon l'invention on utilise, à titre d'agent(s) actif(s) anti-adipeux, exclusivement un ou plusieurs extrait(s) non aqueux de bulbes d'Allium sativum.

Avantageusement et selon l'invention, on utilise entre 3 ppm et 20 ppm d'extrait(s) de bulbes d'Allium sativum.

Avantageusement et selon l'invention, on utilise, à titre d'agent actif anti-adipeux, au moins une huile essentielle et au moins un agent émulsionnant. En variante, avantageusement et selon l'invention qu'on utilise, à titre d'agent actif anti-adipeux, au moins une absolue en solution aqueuse. Avantageusement et selon l'invention on utilise, à titre d'agent actif non-adipeux, au moins un extrait de bulbes d'Allium sativum choisi parmi une huile essentielle ; une absolue obtenue à partir d'une concrète extraite à l'hexane ; une absolue obtenue à partir d'une concrète extraite à l'acétate d'éthyle ; une absolue obtenue à partir d'une oléorésine d'extraction extraite à l'acétone.

Avantageusement, dans un procédé ou une utilisation selon l'invention, on utilise une composition selon l'invention. L'invention concerne aussi plus généralement une composition adaptée pour la mise en oeuvre d'un procédé de traitement (cosmétique et/ou thérapeutique) ou d'un procédé d'inhibition selon l'invention.

L'invention concerne aussi une composition, un procédé de traitement -notamment cosmétique-, un procédé de préparation d'une composition thérapeutique, une utilisation, caractérisés en combinaison par tout ou partie des caractéristiques mentionnées ci-dessus ou ci-après.

L'efficacité de l'invention ressort des résultats des exemples 1 à 4 décrits ci-après et illustrés en référence aux figures 1A à 1F, 2A et 2B, 3A et 3B, annexées :
- les figures 1A à 1F, 2A et 2B sont des vues photographiques microscopiques de cellules adipeuses.
- Les figures 3A et 3B sont des vues photographiques de gels d'électrophorèse.

Les exemples 1, 2, 3 et 4 illustrent l'efficacité biologique sur les processus d'hyperplasie et d'hypertrophie adipocytaires d'une huile essentielle de bulbes d'Allium sativum (dite HE) ; d'une absolue de bulbes d'Allium sativum obtenue à partir d'une concrète extraite à l'hexane (dite ACH) ; d'une absolue de bulbes d'Allium sativum obtenue à partir d'une concrète extraite à l'acétate d'éthyle (dite ACAE) ; d'une absolue de bulbes d'Allium sativum obtenue à partir d'une oléorésine d'extraction extraite à l'acétone (dite AOA). Les modalités de préparation des solutions HE, ACH, ACAE et AOA, les modèles cellulaires utilisés, ainsi que les modalités de traitement sont détaillés ci-dessous.

### Préparation des solutions HE, ACH, ACAE et AOA :

### • Préparations de la solution de HE :

Une solution de HE introduite dans le milieu de culture est préparée en diluant au 1/10 dans de l'eau pour culture cellulaire (Eurobio®, France), une solution mère de HE (commercialisée par SIGMA-ALDRICH FINE CHEMICALS ; "Garlic oil, Chinese", réf. W250309) constituée de 18,4 µl de HE et de 981,6 µl de Liposol (émulsionnant naturel à base de substances extraites de membranes de cellules végétales, commercialisé par SIEGEN, Suisse). Une solution témoin introduite dans le milieu de culture est préparée en diluant au 1/10 dans de l'eau pour culture cellulaire (Eurobio®, France), une solution mère témoin constituée de 18,4 µl d'eau pour culture cellulaire (Eurobio®, France) et de 981,6 µl de Liposol.

### • Préparations des solutions aqueuses absolues ACH, ACAE, et AOA :

Les absolues selon l'invention sont préparées selon un procédé qui en lui-même est classique, bien connu et bien défini dans l'industrie de la parfumerie. Schématiquement, une absolue est un extrait résultant d'au moins deux étapes d'extraction. La première étape est effectuée avec un solvant d'extraction volatil non aqueux -notamment non benzénique- et permet d'obtenir une concrète lorsque le matériel végétal de départ est frais ou décongelé (après avoir été congelé pour sa conservation), ou une oléorésine lorsque ledit matériel est utilisé à l'état sec. La deuxième étape est effectuée avec l'éthanol à titre de solvant. Les absolues ACH, ACAE et AOA sont obtenues par un procédé appliqué sur des bulbes d'Allium sativum .

Pour l'obtention d'une absolue ACH, des gousses d'ail frais sont débarrassées de la peau, puis broyées mécaniquement.

Une extraction à l'hexane est alors effectuée. Par chauffage modéré, sans porter à ébullition, et après totale évaporation de l'hexane, la concrète est récupérée. Celle-ci contient les cires et les produits aromatiques. A température ambiante, elle présente un aspect pâteux de couleur jaune.

Après dissolution de cette concrète dans l'éthanol par lavages, la solution est filtrée puis glacée à -10°C, pour éliminer les cires. Après distillation de l'éthanol, l'absolue ACH est obtenue. Diluée à 25% dans l'éthanol, elle se présente sous l'aspect d'une solution limpide jaune clair, de forte odeur.

L'ACAE est préparée selon un protocole comparable à celui de l'obtention de l'ACH, la première étape consistant cette fois en une extraction à l'acétate d'éthyle.

L'absolue ACAE, diluée à 25% dans l'éthanol, est une solution limpide marron clair, d'odeur moyenne.

L'AOA est préparée selon un protocole comparable à celui de l'obtention de l'ACH, la première extraction se faisant cette fois avec de l'acétone, à partir de gousses d'ail préalablement soumises à une étape de séchage. A l'issue de cette première extraction, une oléorésine d'extraction est obtenue. A température ambiante, elle est sous la forme d'une pâte de couleur marron très foncé.

Celle-ci subit alors une distillation à l'éthanol pour obtenir en final l'absolue AOA.

Diluée à 25% dans l'éthanol, l'absolue AOA se présente sous la forme d'une solution limpide de couleur marron, peu odorante.

Les solutions de ACH, ACAE et AOA introduites dans le milieu de culture sont préparées en diluant, au 1/10 dans de l'eau pour culture cellulaire (Eurobio®, France), des solutions mères de chaque absolue (ACH, ACAE ou AOA) constituées de 18,4 µl de l'absolue (ACH, ACAE ou AOA) et de 981,6 µl d'eau pour culture cellulaire (Eurobio®, France).

### Modèles cellulaires utilisés :

Les mécanismes impliqués dans le processus de différentiation adipocytaire ont été étudiés depuis de nombreuses années *in vitro* (revue générale : Klaus S., BioEssays, 19 :215-223, 1997).

Les activités biologiques de HE, ACH, ACAE et AOA sont mises en évidence à partir de cultures de cellules 3T3-F442A (lignée cellulaire d'origine murine, utilisée pour sa capacité à accumuler des lipides), puis confirmées sur des cultures primaires de préadipocytes humains.

Les cellules sont ensemencées dans des plaques 6 puits (densité d'ensemencement : 15000 cellules/puits) et entretenues jusqu'à confluence en milieu DMEM-10% SVD et antibiotiques. A confluence, les préadipocytes sont alors cultivés en milieu DMEM-10% SVF et insuline. Les cellules traitées sont entretenues dans le milieu de culture auquel on ajoute au final 25 µl de l'une des solutions à tester (HE, ou ACH; ou ACAE, ou AOA) par millilitre de milieu de culture. Les préadipocytes 3T3-F442A témoins sont cultivés dans le milieu de culture auquel on ajoute 25 µl de solution témoin par millilitre de milieu de culture. Le milieu de culture est renouvelé toutes les 48 heures.

### Primo-cultures de Préadipocytes humains :

Les préadipocytes humains (ZenBio®, USA), sont cultivés conformément aux instructions du fournisseur. A réception, les préadipocytes humains sont cultivés dans le milieu adéquat fourni, auquel on ajoute au final de 3 µl à 10 µl de l'une des solutions à tester (HE, ou ACH, ou ACAE, ou AOA) par millilitre de milieu de culture. Les préadipocytes témoins sont maintenus en culture dans le milieu seul. Le milieu de culture est renouvelé toutes les 3 jours.

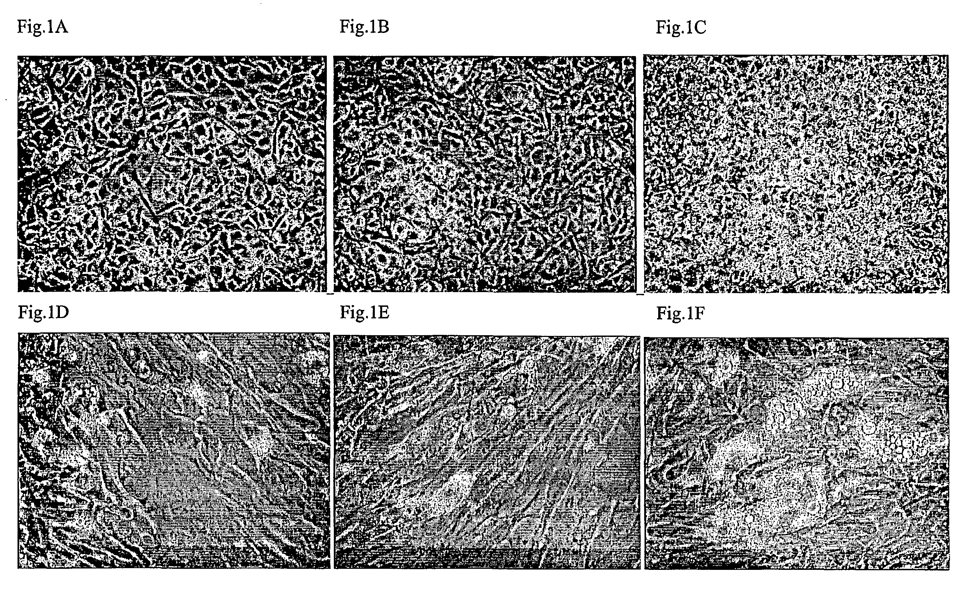

EXEMPLE 1 : Effet des solutions de HE, ACH, ACAE ou AOA sur la morphologie adipocytaire (figures 1A à 1F).

### Au terme de 7 jours (cellules 3T3-F442A) et de 9 jours (pré-adipocytes humains) de traitement l'effet des solutions de HE, ACH, ACAE et AOA sur le processus de différenciation a été étudié sur la base de critères morphologiques, par observation en microscopie des cultures traitées et des cultures témoins. Les cellules sont considérées comme différenciées par analyse morphologique en microscopie phase inverse couplée à une caméra CCD, lorsqu'elles acquièrent un contour rond, et que leur cytoplasme est totalement rempli avec des gouttelettes lipidiques. On constate que les solutions de HE, ACH, ACAE ou AOA inhibent la différenciation cellulaire des préadipocytes murins 3T3-F442A (figures 1A à 1C).

Au terme de 7 jours de traitement chronique les solutions de HE (figure 1A), ou ACH (figure 1B), induisent une diminution significative du nombre d'adipocytes matures développés. La majorité des cellules gardent leur morphologie fibroblastique préadipocytaire avec une très nette inhibition de l'accumulation lipidique. Bien que non illustrés sur les figures, les mêmes résultats sont obtenus avec les solutions de ACAE et de AOA. Les cellules 3T3-F442A témoins (figure 1C), suivent le processus normal de différenciation terminale et acquièrent les caractéristiques morphologiques d'adipocytes matures, avec un cytoplasme rempli de gouttelettes de triglycérides, comme en témoigne la forte réfringence.

On constate aussi que les solutions de HE, ACH, ACAE ou AOA inhibent le processus de différenciation terminale des préadipocytes humains.

Les préadipocytes humains cultivés en présence d'une solution de HE (figure 1D), ou d'une solution de ACH (figure 1E) gardent une morphologie pré-adipocytaire : les cellules humaines restent fusiformes avec très peu de gouttelettes lipidiques intra-cytoplasmiques détectables. Bien que non illustrés sur les figures, les mêmes résultats sont obtenus avec les solutions de ACAE et de AOA. Comparativement, les préadipocytes humains témoins (figure 1F) perdent leur morphologie fibroblastique, avec apparition d'une forme sphérique et l'existence de nombreuses vésicules lipidiques intra-cytoplasmiques-identifiables par leur forte réfringence.

Le traitement chronique par l'huile essentielle de bulbes d'Allium sativum (HE), l'absolue de bulbes d'Allium sativum obtenue à partir d'une concrète extraite à l'hexane (ACH), l'absolue de bulbes d'Allium sativum obtenue à partir d'une concrète extraite à l'acétate d'éthyle (ACAE), l'absolue de bulbes d'Allium sativum obtenue à partir d'une oléorésine d'extraction extraite à l'acétone (AOA) limite les changements morphologiques et biochimiques caractéristiques de la différenciation adipocytaire. Au contact de HE, ACH, ACAE et AOA les préadipocytes ne sont plus sensibles à l'environnement hormonal qui régule leur métabolisme lipidique et qui permet normalement la conversion des préadipocytes en cellules adipeuses différenciées matures.

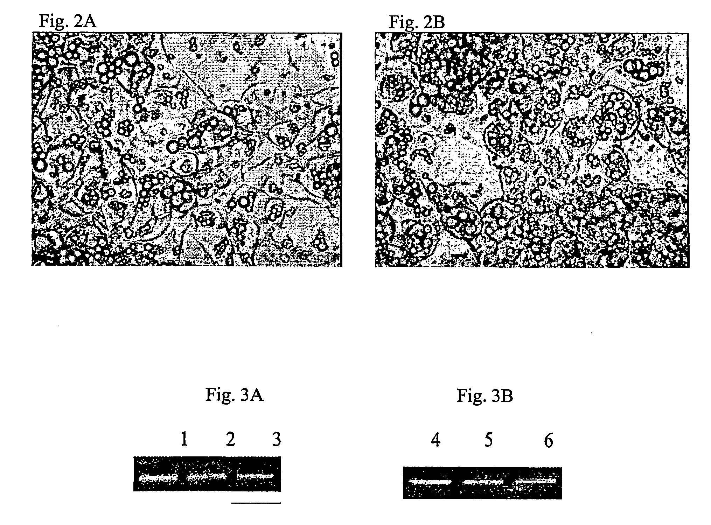

### EXEMPLE 2 : Effet des solutions de HE, ACH, ACAE ou AOA sur la morphologie d'adipocytes hypertrophiés matures (figures 2A et 2B).

A confluence, les préadipocytes 3T3-F442A sont cultivés durant 4 jours en milieu différenciant (DMEM-10% SVF et insuline). Dans ces conditions, les cellules passent au stade de « adipocytes matures » dont le volume et le contenu en triglycérides a été augmenté. Les cellules sont alors traitées comme précédemment.

Comparativement aux adipocytes matures témoins (figure 2B), qui ont une forme volumineuse sphérique et une accumulation importante de vésicules lipidiques intra-cytoplasmiques, les adipocytes matures traités par une solution de HE (figure 2A), ont une morphologie cellulaire moins arrondie et un contenu en vésicules lipidiques intra-adipocytaires fortement diminué, de sorte que la solution de HE, selon l'invention s'avère efficace dans le processus de limitation de l'hypertrophie adipocytaire, en inversant le processus de différenciation. Les mêmes résultats sont obtenus avec les solutions de ACH, ou ACAE ou AOA selon l'invention.

### EXEMPLE 3 : Effet des solutions de HE, ACH, ACAE ou AOA sur l'expression de l'ARNm de PPARγ2 (figures 3A et 3B).

La différenciation des adipocytes est une composante importante du développement du tissu adipeux et de l'obésité. Le processus de différenciation adipocytaire est caractérisé in vitro par l'induction programmée de différents gènes régulant la lipolyse lipoprotéique, le captage cellule des acides gras, et la synthèse des acides gras et des triglycérides. Schématiquement, ces gènes peuvent être classés comme marqueurs de différenciation de type : très précoces (tel que l'expression de l'ARNm de la lipoprotéine lipase), précoces (tel que l'expression de l'ARNm de PPARγ2) ou tardifs (tel que l'expression de la leptine) (revue générale : Morrison R.F., Farmer S.R., J. Cell. Biochem Suppl. 32/33 : 59-67, 1999). Ainsi, le stade de différenciation des adipocytes peut être établi précisément en étudiant le niveau d'expression des ARNm pertinents (ARNm de PPARγ2) ou par le niveau d'expression de la leptine.

La famille des récepteurs nucléaires de type PPAR ("Peroxisomal Proliferator-Activated Receptor") est constituée de 3 sous-types : PPARα, PPARδ, PPARγ. PPARα est fortement exprimé dans le foie, il y régule l'expression des gènes impliqués dans le métabolisme lipidique. L'expression de PPARδ est ubiquitaire ; la fonction de ce récepteur reste encore à déterminer. PPARγ existe sous la forme de deux isoformes PPARγ et PPARγ2. C'est PPARγ2 qui est fortement exprimé au niveau du tissu adipeux des mammifères. Lorsque PPARγ2 est activé, il induit la transcription de plusieurs gènes adipocytaires codant pour des protéines et des enzymes impliquées dans la création et le maintien du phénotype adipocytaire. Il joue donc un rôle capital dans la différenciation et le métabolisme des cellules adipeuses.

L'effet sur l'expression de l'ARNm de PPARγ2 des solutions de : HE, ACH, ACAE ou AOA a été déterminé au terme de 3 jours (figure 3A, cellules 3T3-F442A) ou de 6 jours (figure 3B, pré-adipocytes humains) de traitement. Le contenu en ARNm PPARγ2 est analysé par RT-PCR ("Reverse Transcription-Polymerase Chain Reaction"), après avoir préalablement : purifié l'ARN conformément à la procédure du kit RNeasy Total RNA System (Qiagen®), quantifié la teneur en ARN des échantillons par la mesure et le rapport des absorbances à 260 et 280 nm, et vérifié après migration sur gel d'agarose la qualité des ARN obtenus.

Pour l'étape RT, l'ADN complémentaire (ADNc), est synthétisé à partir de 1 µg d'ARN total (3T3-F442A) ou de 0,2 µg d'ARN total (pré-adipocytes humains). Les amplifications de l'ADNc codant pour PPARγ2 sont obtenues en présence des amorces Sens et Anti-sens adéquates (Genset®, cf. Tableau 1). Les tailles des produits de PCR sont de 307 paires de bases (cellules 3T3-F442A) et de 582 paires de bases (préadipocytes humains). Les contrôles adéquats (étapes de reverse transcription et amplification par PCR) ont été réalisés. Les mélanges de PCR ont été soumis à 22 cycles (cellules 3T3-F442A) ou 35 cycles (préadipocytes humains) d'amplification par dénaturation (2 minutes à 94°C), hybridation (1 minute à 60°C) et élongation (6 minutes à 72°C). Les produits de PCR sont analysés par électrophorèse en gel d'agarose et visualisés au bromure d'éthidium.

**Tableau 1**

| | PPARγ2 murin | PPARγ2 humain |
|---|---|---|
| Sens | 5'-TGTTGACCCAGAGCATGGTGCCT-3' | 5'-GCGATTCCTTCACTGATAC-3' |
| Anti-sens | 5'-CAGGTTCTACTTTGATCGCACTT-3' | 5'-GCATTATGAGACATCCCCAC-3' |

Le niveau d'expression du facteur de transcription adipogénique PPARγ2 est fortement diminué lorsque les cellules 3T3-F442A ont été au contact d'une solution de HE (figure 3A, résultat 2), d'une solution de ACH (figure 3A, résultat 3), comparativement aux cellules témoins comme en témoigne l'intensité de la bande (figure 3A, résultat 1). Les mêmes résultats sont obtenus avec la solution de ACAE et la solution de AOA.

De même, les solutions de HE, ACH, ACAE ou AOA selon l'invention inhibent significativement l'expression de l'ARNm PPARγ2 des préadipocytes humains (figure 3B, le résultat 5 illustrant un traitement avec une solution de HE, et le résultat 6 illustrant un traitement avec une solution de ACH), comparativement aux préadipocytes humains témoins (figure 3B, résultat 4).

L'huile essentielle de bulbes d'Allium sativum (HE), l'absolue de bulbes d'Allium sativum obtenue à partir d'une concrète extraite à l'hexane (ACH), l'absolue de bulbes d'Allium sativum obtenue à partir d'une concrète extraite à l'acétate d'éthyle (ACAE), l'absolue de bulbes d'Allium sativum obtenue à partir d'une oléorésine d'extraction extraite à l'acétone (AOA), agissent au niveau du programme de différenciation en diminuant l'expression du messager de PPARγ2 .

### EXEMPLE 4 : Effet de HE, ACH, ACAE et AOA sur la sécrétion de leptine.

La capacité de HE, ACH, ACAE et AOA à inhiber le processus de différenciation des préadipocytes humains a été déterminée par la mesure quantitative des taux de leptine sécrétée dans le milieu de culture des différents essais. En effet, la leptine codée par le gène *ob,* synthétisée et sécrétée par les adipocytes, est considérée comme étant un marqueur tardif du processus de différentiation adipocytaire. *In vitro,* la sécrétion basale de leptine augmente progressivement tout au long du processus de conversion des préadipocytes en adipocytes matures (MacDougald, O.A. et al, Proc Natl Acad Sci, USA, 92 : 9034-9037, 1995).

L'effet sur la sécrétion de la leptine des solutions de HE, ACH, ACAE et AOA a été étudié sur une période de traitement de 9 jours (préadipocytes humains). Les taux de leptine sécrétée par des cellules témoins et par des cellules traitées ont été mesurés dans les différents milieux de culture, prélevés à 3, 6, et 9 jours (jours de renouvellement du milieu). La détermination quantitative des taux de leptine libérée est réalisé par dosage ELISA (R&D Systems Europe), conformément aux instructions du fournisseur. Les résultats sont exprimés en moyenne ± écart type à la moyenne. Les comparaisons statistiques sont réalisées par le test T de Student (p<0,05 représentant le seuil de significativité).

Les résultats sont normalisés comme suit : au terme des 9 jours de traitement, les valeurs en leptine quantifiées pour chacun des traitements au troisième, au sixième et au neuvième jours sont cumulées et exprimées en pourcentage des valeurs obtenues pour les cellules témoins (Tableau 2). Tableau 2 - Effet de HE, ACH, ACAE ou AOA sur la sécrétion de leptine

| Traitement | Concentration | Leptine (%) | | Significativité |
|---|---|---|---|---|
| | | Moyenne | Ecart Type à la moyenne | |
| HE | 14 ppm | 69,7 | 2,7 | P<0,001 |
| ACH | 3 ppm | 69,4 | 4,4 | P<0,01 |
| ACH | 10 ppm | 36,3 | 0,1 | P<0,001 |
| ACAE | 10 ppm | 81,7 | 2,1 | P<0,01 |
| AOA | 10 ppm | 80,9 | 2,1 | P<0,001 |

Les préadipocytes humains cultivés en présence de HE, ACH, ACAE et AOA sécrètent dans le milieu de culture des quantités de leptine significativement inférieures à celles détectées dans le milieu de culture des préadipocytes humains témoins non traités. Les résultats obtenus démontrent que les solutions de HE, ACH, ACAE ou AOA agissent au niveau du programme de différenciation et sont capables d'inhiber le processus de conversion adipocytaire.

## Revendications

1. Composition de traitement topique à usage externe **caractérisée en ce qu'**elle est constituée :
. d'une quantité efficace, correspondant à une proportion pondérale de 3 à 20 ppm de la composition totale, d'au moins un agent actif anti-adipeux choisi parmi les extraits de bulbes d'Allium sativum à l'exception d'une oléorésine d'extraction extraite à l'hexane,
. optionnellement, d'un ou plusieurs autres agents actifs complémentaires, à l'exception des extraits de Nympheaceae,
. d'un excipient cosmétiquement ou pharmaceutiquement acceptable et adapté pour une application topique à usage externe -notamment sur la peau.

2. Composition selon la revendication 1, **caractérisée en ce qu'**elle comprend une quantité efficace d'au moins un agent actif anti-adipeux choisi parmi les extraits non aqueux de bulbes d'Allium sativum.

3. Composition selon l'une des revendications 1 ou 2, **caractérisée en ce qu'**elle comprend, à titre d'agent actif anti-adipeux, une huile essentielle de bulbes d'Allium sativum et un agent émulsionnant.

4. Composition selon la revendication 3, **caractérisée en ce que** le mélange huile essentielle / agent émulsionnant est incorporé dans un gel et un liquide tel que l'eau.

5. Composition selon l'une des revendications 1 à 4, **caractérisée en ce qu'**elle comprend, à titre d'agent actif anti-adipeux, au moins une absolue de bulbes d'Allium sativum en solution aqueuse.

6. Composition selon la revendication 5, **caractérisée en ce qu'**elle comprend au moins une absolue obtenue à partir d'une concrète extraite de bulbes d'Allium sativum.

7. Composition selon la revendication 6, **caractérisée en ce qu'**elle comprend en outre au moins une absolue obtenue à partir d'une oléorésine d'extraction extraite de bulbes d'Allium sativum.

8. Composition selon l'une des revendications 1 à 7,
**caractérisée en ce qu'**elle comprend, à titre d'agent actif anti-adipeux, au moins un extrait de bulbes d'Allium sativum choisi parmi une huile essentielle, une absolue obtenue à partir d'une concrète extraite à l'hexane, une absolue obtenue à partir d'une concrète extraite à l'acétate d'éthyle, une absolue obtenue à partir d'une oléorésine d'extraction extraite à l'acétone.

9. Composition selon l'une des revendications 1 à 8, **caractérisée en ce que** le(s) extrait(s) de bulbes d'Allium sativum constitue(nt) le(s) seul(s) agent(s) actif(s) anti-adipeux.

10. Composition selon l'une des revendications 1 à 9, **caractérisée en ce qu'**elle est exempte d'agent actif complémentaire.

11. Composition selon l'une des revendications 1 à 9, **caractérisée en ce qu'**elle comprend au moins un agent lipolytique à titre d'agent actif complémentaire.

12. Procédé de traitement cosmétique préventif ou curatif de la cellulite et des surcharges adipeuses dermiques localisées, **caractérisé en ce qu'**on applique par voie topique sur des zones de peau à traiter une composition contenant au moins un extrait de bulbe d'Allium sativum à titre d'agent actif; la quantité d'extrait de bulbes d'Allium sativum de ladite composition thérapeutique correspond à une proportion pondérale de 3 à 20 ppm de la composition totale.

13. Procédé de traitement cosmétique préventif ou curatif de la cellulite et des surcharges adipeuses dermiques localisées, **caractérisé en ce qu'**on utilise une composition selon l'une des revendications 1 à 11.

14. Utilisation d'au moins un extrait de bulbes d'Allium sativum à titre d'agent actif anti-adipeux pour la préparation d'une composition thérapeutique pour le traitement préventif ou curatif, par application topique sur des zones de peau à traiter, de l'obésité ; la quantité d'extrait de bulbes d'Allium sativum de ladite composition thérapeutique correspond à une proportion pondérale de 3 à 20 ppm de la composition totale.

15. Procédé de préparation d'une composition thérapeutique pour le traitement préventif ou curatif, par application topique sur des zones de peau à traiter, de l'obésité, **caractérisé en ce qu'**on incorpore dans la composition une quantité efficace, correspondant à une proportion pondérale de 3 à 20 ppm de la composition totale, d'au moins un extrait de bulbes d'Allium sativum à titre d'agent actif anti-adipeux.

16. Procédé d'inhibition extra-corporelle de la conversion adipocytaire des préadipocytes en adipocytes matures, **caractérisé en ce qu'**on met une composition selon l'une des revendications 1 à 11 au contact des préadipocytes ou d'un tissu biologique les incorporant.

17. Utilisation ou procédé selon l'une des revendications 12 à 16, **caractérisé en ce qu'**on utilise, à titre d'agent(s) actif(s) anti-adipeux exclusivement un ou plusieurs extrait(s) non aqueux de bulbes d'Allium sativum.

18. Utilisation ou procédé selon l'une des revendications 12 à 16, **caractérisée en ce qu'**on utilise, à titre d'agent actif anti-adipeux, au moins une huile essentielle et au moins un agent émulsionnant.

19. Utilisation ou procédé selon l'une des revendications 12 à 16, **caractérisée en ce qu'**on utilise, à titre d'agent actif anti-adipeux, au moins une absolue en solution aqueuse.

20. Utilisation ou procédé selon l'une des revendications 12 à 19, **caractérisée en ce qu'**on utilise, à titre d'agent actif non-adipeux, au moins un extrait de bulbes d'Allium sativum choisi parmi une huile essentielle ; une absolue obtenue à partir d'une concrète extraite à l'hexane ; une absolue obtenue à partir d'une concrète extraite à l'acétate d'éthyle ; une absolue obtenue à partir d'une oléorésine d'extraction extraite à l'acétone.

## Claims

1. Topical treatment composition for external use, **characterized in that** it consists:
• of an effective amount, corresponding to a weight proportion of 3 to 20 ppm of the total composition, of at least one anti-adipose active agent chosen from extracts of Allium sativum bulbs, with the exception of an oleoresin extracted with hexane,
• optionally, of one or more other additional active agents with the exception of Nymphaceae extracts,
• of a cosmetically or pharmaceutically acceptable excipient that is suitable for topical application for external use - especially on the skin.

2. Composition according to Claim 1, **characterized in that** it comprises an effective amount of at least one anti-adipose active agent chosen from non-aqueous extracts of Allium sativum bulbs.

3. Composition according to either of Claims 1 to 2, **characterized in that** it comprises, as anti-adipose active agent, an essential oil of Allium sativum bulbs and an emulsifier.

4. Composition according to Claim 3, **characterized in that** the essential oil/emulsifier mixture is incorporated into a gel and a liquid such as water.

5. Composition according to one of Claims 1 to 4, **characterized in that** it comprises, as anti-adipose active agent, at least one absolute of Allium sativum bulbs in aqueous solution.

6. Composition according to claim 5, **characterized in that** it comprises at least one absolute obtained from a concrete extracted from Allium sativum bulbs.

7. Composition according to Claim 6, **characterized in that** it also comprises at least one absolute obtained from an oleoresin extracted from Allium sativum bulbs.

8. Composition according to one of Claims 1 to 7, **characterized in that** it comprises, as anti-adipose active agent, at least one extract of Allium sativum bulbs chosen from an essential oil, an absolute obtained from a concrete extracted with hexane, an absolute obtained from a concrete extracted with ethyl acetate, and an absolute obtained from an oleoresin extracted with acetone.

9. Composition according to one of Claims 1 to 8, **characterized in that** the extract(s) of Allium sativum bulbs constitute(s) the only anti-adipose active agent(s).

10. Composition according to one of Claims 1 to 9, **characterized in that** it is free of additional active agent.

11. Composition according to one of Claims 1 to 9, **characterized in that** it comprises at least one lipolytic agent as additional active agent.

12. Preventive or curative cosmetic process for treating cellulite and localized dermal excess adipose, **characterized in that** a composition containing at least one extract of Allium sativum bulbs is applied topically as an active agent to areas of skin to be treated; the quantity of Allium sativum bulb extract of the said therapeutic composition corresponds to a weight proportion of 3 to 20 ppm of the total composition.

13. Preventive or curative cosmetic process for treating cellulite and localized dermal excess adipose, **characterized in that** a composition according to one of Claims 1 to 11 is used.

14. Use of at least one extract of Allium sativum bulbs as an anti-adipose active agent for the preparation of a therapeutic composition for the preventive or curative treatment of obesity, by topical application to areas of skin to be treated; the quantity of Allium sativum bulb extract of the said therapeutic composition corresponds to a weight proportion of 3 to 20 ppm of the total composition.

15. Process for preparing a therapeutic composition for the preventive or curative treatment of obesity, by topical application to areas of skin to be treated, **characterized in that** an effective amount, corresponding to a weight proportion of 3 to 20 ppm of the total composition of at least one extract of Allium sativum bulbs is incorporated into the composition as an anti-adipose active agent.

16. Process for the extra-corporeal inhibition of the adipocytary conversion of preadipocytes into mature adipocytes, **characterized in that** a composition according to one of claims 1 to 11 is put into contact with preadipocytes or a biological tissue incorporating these.

17. Use or process according to one of Claims 12 to 16, **characterized in that** one or more non-aqueous extract(s) of Allium sativum bulbs is (are) used exclusively as anti-adipose active agent(s).

18. Use or process according to one of Claims 12 to 16, **characterized in that** at least one essential oil and at least one emulsifying agent is used as an anti-adipose active agent.

19. Use or process according to one of Claims 12 to 16, **characterized in that** at least one absolute in aqueous solution is used as an anti-adipose active agent.

20. Use or process according to one of Claims 12 to 19, **characterized in that** at least one extract of Allium sativum bulbs chosen from an essential oil; an absolute obtained from a concrete extracted with hexane; an absolute obtained from a concrete extracted with ethyl acetate; and an absolute obtained from an oleoresin extracted with acetone, is used as non-adipose active agent.

## Patentansprüche

1. Zusammensetzung zur topischen externen Behandlung, **dadurch gekennzeichnet, dass** sie Folgendes umfasst:
. eine wirksame Menge, die einem Gewichtsanteil von 3 bis 20 ppm der Gesamtzusammensetzung entspricht, von wenigstens einem antiadipösen Wirkstoff, der aus Extrakten von Allium sativum, mit Ausnahme von einem mit Hexan extrahierten Extraktionsoleoresin, ausgewählt ist;
. bei Bedarf ein oder mehrere weitere komplementäre Wirkstoffe, mit Ausnahme von Nympheaceae-Extrakten,
. ein kosmetisch oder pharmazeutisch akzeptables Bindemittel, das äußerlich - insbesondere auf die Hauttopisch angewendet werden kann.

2. Zusammensetzung nach Anspruch 1, **dadurch gekennzeichnet, dass** sie eine wirksame Menge von wenigstens einem antiadipösen Wirkstoff umfasst, der aus nichtwässrigen Extrakten von Allium sativum ausgewählt ist.

3. Zusammensetzung nach Anspruch 1 oder 2, **dadurch gekennzeichnet, dass** sie als antiadipösen Wirkstoff ein essentielles Öl von Allium sativum und einen Emulgator umfasst.

4. Zusammensetzung nach Anspruch 3, **dadurch gekennzeichnet, dass** das Gemisch aus essentiellem Öl und Emulgator in einem Gel und einer Flüssigkeit wie Wasser integriert ist.

5. Zusammensetzung nach einem der Ansprüche 1 bis 4, **dadurch gekennzeichnet, dass** sie als antiadipösen Wirkstoff wenigstens ein absolutes Öl von Allium sativum in wässriger Lösung umfasst.

6. Zusammensetzung nach Anspruch 5, **dadurch gekennzeichnet, dass** sie wenigstens ein absolutes Öl umfasst, das auf der Basis eines aus Allium sativum extrahierten Concrete erhalten wurde.

7. Zusammensetzung nach Anspruch 6, **dadurch gekennzeichnet, dass** sie darüber hinaus wenigstens ein absolutes Öl umfasst, das auf der Basis eines aus Allium sativum extrahierten Extraktionsoleoresins erhalten wurde.

8. Zusammensetzung nach einem der Ansprüche 1 bis 7, **dadurch gekennzeichnet, dass** sie als antiadipösen Wirkstoff wenigstens einen Extrakt von Allium sativum umfasst, ausgewählt aus einem essentiellen Öl, einem absoluten Öl, das auf der Basis eines mit Hexan extrahierten Concrete erhalten wurde, einem absoluten Öl, das auf der Basis eines mit Ethylacetat extrahierten Concrete erhalten wurde, und einem absoluten Öl, das auf der Basis eines mit Aceton extrahierten Extraktionsoleoresins erhalten wurde.

9. Zusammensetzung nach einem der Ansprüche 1 bis 8, **dadurch gekennzeichnet, dass** der/die Extrakt(e) von Allium sativum den/die einzigen antiadipösen Wirkstoff(e) bildet/bilden.

10. Zusammensetzung nach einem der Ansprüche 1 bis 9, **dadurch gekennzeichnet, dass** sie keinen komplementären Wirkstoff aufweist.

11. Zusammensetzung nach einem der Ansprüche 1 bis 9, **dadurch gekennzeichnet, dass** sie wenigstens ein lipolytisches Mittel als komplementären Wirkstoff umfasst.

12. Verfahren zur präventiven oder kurativen kosmetischen Behandlung von Cellulit und von lokalisierten dermalen adipösen Überlastungen, **dadurch gekennzeichnet, dass** auf topischem Weg auf die zu behandelnden Hautzonen eine Zusammensetzung aufgetragen wird, die wenigstens einen Extrakt von Allium sativum als Wirkstoff enthält, wobei die Menge des Extrakts von Allium sativum der genannten therapeutischen Zusammensetzung einem Gewichtsanteil von 3 bis 20 ppm der Gesamtzusammensetzung entspricht.

13. Verfahren zur präventiven oder kurativen kosmetischen Behandlung von Cellulit und von lokalisierten dermalen adipösen Überlastungen, **dadurch gekennzeichnet, dass** eine Zusammensetzung nach einem der Ansprüche 1 bis 11 verwendet wird.

14. Verwendung von wenigstens einem Extrakt von Allium sativum als antiadipösen Wirkstoff für die Herstellung einer therapeutischen Zusammensetzung für die präventive oder kurative Behandlung von Fettleibigkeit durch topische Anwendung auf die zu behandelnden Hautzonen, wobei die Menge des Extrakts von Allium sativum der genannten therapeutischen Zusammensetzung einem Gewichtsanteil von 3 bis 20 ppm der Gesamtzusammensetzung entspricht.

15. Verfahren zur Herstellung einer therapeutischen Zusammensetzung für die präventive oder kurative Behandlung, durch topische Applikation auf zu behandelnde Hautzonen, von Fettleibigkeit, **dadurch gekennzeichnet, dass** in die Zusammensetzung eine wirksame Menge, die einem Gewichtsanteil von 3 bis 20 ppm der Gesamtzusammensetzung entspricht, von wenigstens einem Extrakt von Allium sativum als antiadipösen Wirkstoff integriert wird.

16. Verfahren zum extrakorporalen Hemmen der adipozytären Umwandlung von Präadipozyten in reife Adipozyten, **dadurch gekennzeichnet, dass** eine Zusammensetzung nach einem der Ansprüche 1 bis 11 mit Präadipozyten oder einem sie enthaltenden biologischen Gewebe in Kontakt gebracht wird.

17. Verwendung oder Verfahren nach einem der Ansprüche 12 bis 16, **dadurch gekennzeichnet, dass** als antiadipöse(r) Wirkstoff(e) ausschließlich ein oder mehrere nichtwässrige Extrakte von Allium sativum verwendet werden.

18. Verwendung oder Verfahren nach einem der Ansprüche 12 bis 16, **dadurch gekennzeichnet, dass** als antiadipöser Wirkstoff wenigstens ein essentielles Öl und wenigstens ein Emulgator verwendet werden.

19. Verwendung oder Verfahren nach einem der Ansprüche 12 bis 16, **dadurch gekennzeichnet, dass** als antiadipöser Wirkstoff wenigstens ein absolutes Öl in wässriger Lösung verwendet wird.

20. Verwendung oder Verfahren nach einem der Ansprüche 12 bis 19, **dadurch gekennzeichnet, dass** wenigstens ein Extrakt von Allium sativum, der aus einem essentiellen Öl ausgewählt wurde; ein absolutes Öl, das auf der Basis eines mit Hexan extrahierten Concrete erhalten wurde; ein absolutes Öl, das auf der Basis eines mit Ethylacetat extrahierten Concrete erhalten wurde; ein absolutes Öl, das auf der Basis eines mit Aceton extrahierten Extraktionsoleoresins erhalten wurde, als nichtadipöser aktiver Wirkstoff verwendet wird.
